(19) Europäisches Patentamt
European Patent Office
Office européen des brevets

(11) Publication number: 0 247 971
A2

(12) # EUROPEAN PATENT APPLICATION

(21) Application number: 87810305.0

(51) Int. Cl.⁴: A 61 K 31/47

(22) Date of filing: 20.05.87

(30) Priority: 29.05.86 GB 8613019

(43) Date of publication of application: 02.12.87
Bulletin 87/49

(84) Designated Contracting States: AT BE CH DE ES FR GB GR IT LI LU NL SE

(71) Applicant: SANDOZ AG, Lichtstrasse 35, CH-4002 Basel (CH)
(84) Designated Contracting States: BE CH ES FR GB GR IT LI LU NL SE

(71) Applicant: SANDOZ-PATENT-GMBH, Humboldtstrasse 3, D-7850 Lörrach (DE)
(84) Designated Contracting States: DE

(71) Applicant: SANDOZ-ERFINDUNGEN Verwaltungsgesellschaft m.b.H., Brunner Strasse 59, A-1235 Wien (AT)
(84) Designated Contracting States: AT

(72) Inventor: Morley, John, Lilienstrasse 64, CH-4124 Allschwil (CH)

(54) **Novel pharmaceutical compositions comprising and use of cis-6-(4-acetanilido)-8,9-dimethoxy-2-methyl-1,2,3,4,4a,10b-hexahydrobenzo[c][1,6]naphthyridin.**

(57) Use of cis-6-(4-acetanilido)-8,9-dimethoxy-2-methyl-1,-2,3,4,4a,10b-hexahydro-benzo[c][1,6]naphthyridine in the treatment of obstructive or inflammatory airways disease and disease characterised by, or having an aetiology comprising, morbid eosinophil accumulation, for example asthma, pneumoconiosis, hypereosinophilia, eosinophil related disorders, atopy and bronchitis, as well as novel pharmaceutical compositions comprising said benzonaphthyridine derivative suitable for such use.

EP 0 247 971 A2

ACTORUM AG

0247971

1

100-6907

NOVEL PHARMACEUTICAL COMPOSITIONS COMPRISING, AND USE OF, CIS-6-
-(4-ACETANILIDO)-8,9-DIMETHOXY-2-METHYL-1,2,3,4,4a,10b-HEXAHYDRO-
BENZO[c][1,6]NAPHTHYRIDIN

The present invention relates to a new use, in particular a new pharmaceutical use for the compound cis-6-(4-acetanilido)-8,9-dimethoxy-2-methyl-1,2,3,4,4a,10b-hexahydro-benzo[c][1,6]naphthyridin, (hereinafter referred to for convenience as "COMPOUND") of formula:

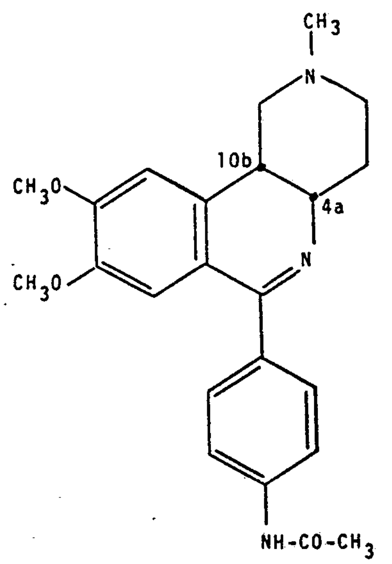

the configuration across carbon atoms 4a and 10b being cis. COMPOUND is known and has been described together with processes for its production, as a member of a broader compound class

e.g. in US Patent No. 3,899,494 (see in particular example 6) and equivalents thereof world-wide including UK Patent Specifications Nos. 1,361,441 and 1,361,442, German Offenlegungsschrift No. 2 123 328 and Danish Patent Specification No. 134 488 (the latter including examples of a number of additional compounds belonging to the same general class, not specifically disclosed in other countries).

Original interest in COMPOUND and other compounds of the general class disclosed in US Patent No. 3,899,494 derived from ability to inhibit blood platelet aggregation (BPA), that is gathering together of blood platelets in adherent masses, e.g. in the presence of adenosine diphosphate and other BPA inducers such as adrenalin and collagen, and hence potential pharmaceutical utility as anti-thrombotic agents [See e.g. U.S. Patent No. 3,899,494 as well as Walker et al. and Livsey et al., both Brit. J. Pharmacol., 43 (2), 460 - 461 (1971) and 55 (2), 258 P - 59 P (1975), COMPOUND being variously referred to in said references and other references cited hereunder by its full chemical name as set forth above, by the code No. AH 21-132 or by the provisional generic name "Benafentrine".] Results for COMPOUND in subsequent clinical investigations in the anti-thrombotic indication were however insufficient to warrant full pharmaceutical development and clinical trials were accordingly discontinued.

Pharmacological investigation of COMPOUND during development as a potential anti-thrombotic agent revealed that it also possesses cyclic adenosine monophosphate (cAMP) phosphodiesterase inhibiting activity. COMPOUND has subsequently found some utility as a research tool in the investigation of the possible rôle played by intraneuronal cAMP accumulation (following cAMP phosphodiesterase blocking) in the regulation of noradrenalin release from e.g. cerebrocortical tissues and sympathetic nerve

fibres, for example as evidenced directly by COMPOUND's ability to enhance induced noradrenalin release from appropriate nerve tissue preparations or, indirectly, by ability to inhibit electrically evoked contraction of pulmonary artery preparations [see e.g. Markstein et. al., Göthert et al. and Hentrich et al., all Naunyn-Schmiedeberg's Arch. Pharmacol.: 325, 17 - 24 (1984); 328, 127 - 134 (1984); and 330, 245 - 244 (1985)].

Having regard to the mooted hypothesis [see e.g. Sutherland et al., Biol. Chem. 237, 1223 (1962)] that cAMP might be directly involved in the contractile function of the heart and act as a mediator of inotropic activity, possible utility of COMPOUND as a stimulant of heart contraction and heamodynamic activity was also investigated. COMPOUND was indeed found to exhibit inotropic activity as well as an ability to reduce blood-pressure via peripheral vasodilation. COMPOUND was accordingly submitted for preliminary clinical investigation in subjects exhibiting chronic heart insufficiency (CHI) [Betschart et al., Zeitschrift für Kardiologie, 68 (4), 280 (1979)]. Despite initial enthusiasm for COMPOUND in the CHI indication, as in the case of the previously mooted anti-thrombotic indication, clinical results proved insufficient to warrant extended clinical development.

In accordance with the present invention it has now unexpectedly been found that COMPOUND is useful both as a bronchodilator drug, e.g. for the symptomatic treatment of obstructive or inflammatory airways disease, and as a drug for the prophylactic treatment of inflammatory airways disease, e.g. for the suppression or diminution of basal pathology of such disease, in particular airways hyperreactivity as well as eosinophil accumulation, and hence advance treatment or prevention of symptomatic attack. COMPOUND is thus, in accordance with the particular findings of

this invention, eminently suited for the treatment of such disease as well as a variety of non-airways related diseases of shared aetiology.

With respect to its bronchodilator efficacy, COMPOUND has in particular been found to be non-selective. That is, it has been found to be capable of suppressing bronchospasm induced by both spasmogens and allergens as well as of reversing sustained bronchospasm induced e.g. by injection or infusion of the peptide bombesin. It has also surprisingly been found that COMPOUND is capable of relaxing airways smooth muscle *in vitro* with equal facility, whether contracted by endogenous tone or by addition of spasmogens. Since COMPOUND is found to have relaxant effects that are independent of contractile stimulus, it is apparent that its bronchodilator efficacy is not associated with selective antagonism of known spasmogens as in the case of established bronchodilator drugs, for example belonging to the methyl-xanthine class (e.g. Theophylline and Aminophylline). ß-Adrenoceptor agonist bronchodilator drugs (or "$ß_2$-agonists", for example Salbutamol) exhibit similar independence of action. The effects of COMPOUND however are not attributable to interaction with the ß-adrenoceptor system, since potency and efficacy of COMPOUND as an airways smooth muscle spasmolytic are found not to be diminished by ß-adrenoceptor blocking agents (e.g. Propanalol) or agents (e.g. Isoprenaline) inducing tachyphylaxis (desensitisation) to $ß_2$-agonist drugs. It thus appears that the mode of action of COMPOUND as a bronchodilator as revealed by the findings of the present invention is essentially novel in kind.

COMPOUND has also been found to exhibit greater bronchodilator potency than other drug substances (e.g. Aminophylline) established for use as bronchodilator agents or previously proposed for use as bronchodilator agents, e.g. in the symptomatic

treatment of asthma.

With respect to utility in the prophylactic treatment of inflammatory airways disease, it has even more surprisingly been found that COMPOUND:

a) inhibits acute response in hypersensitive subjects following allergen or other challenge eliciting hypersensitivity reaction, e.g. following induction of hyperreactivity and airways obstruction via PAF (platelet activating factor) challenge;
b) suppresses development of airways hyperreactivity subsequent to challenge as under a);
c) diminishes basal (or on-going) airways hyperreactivity; and
d) suppresses eosinophil accumulation, e.g. sustained eosinophil accumulation following airways obstruction induced e.g. by PAF challenge.

(For further discussion relevant to these findings and their relationship to prophylactic utility in treating inflammatory airways disease, see e.g.:

1. Altounyan, Clinical Allergy (supp.) 10, 481-489 (1980),

- relationship between reduction of airways hyperreactivity and of occurrence of asthma symptomatology (acute and late-onset airway obstruction) as evidenced by the actions of the known asthma prophylactic drug sodium chromoglycate or SCG;

2.1 Morley et al., Lancet ii, 1142-1144 (1984),
2.2 Mazzoni et al., J. Physiol., 365, 107 P (1985),
2.3 Traietti et al., Respiration, 46, 62-63 (1984),

2.4 Taytard et al., American Review of Respiratory Disease, 134, 983-985 (1986), and
2.5 Szczeklik et al., Thrombosis and Heamostasis, 56, 283-287 (1986),

- the rôle and involvement of PAF in asthma;

3. Morley, "Drug Therapy for Asthma", Eds.J.W. Jenne and K.R. Murphey, Pub. Marcel Dekker, New York (1987),

- involvement of selective eosinophil accumulation in e.g. PAF-induced acute and sustained airways hyperreactivity;

4.1 Basran et al. Clin. Allergy, 14, 75-79 (1984), and
4.2 Karlsson et al. Brit. J. Clin. Pharmacol., 27, 371-374 (1985).

- evidence that the effects of PAF in man are inhibited by the asthma prophylactic drug SCG;

5. Mazzoni et al., Brit. J. Pharmacol., 86, 571 P (1985)

- evidence that all known asthma prophylactic drugs impair the development of acute airways hyperreactivity following PAF challenge, whereas other symptomatic/non-prophylactic anti-asthma (e.g. exclusively bronchodilator or exclusively anti-inflammatory) drug categories are ineffectual in this regard).

The above elucidation and commentary on basic pharmacology pertinant to the present invention is intended for essentially explanatory purposes only, e.g. as an aid to understanding of the particular nature of the invention and the particular import of

examples hereinafter provided. They are not to be understood as imposing any limitation on the scope of the invention, e.g. in terms of the specific mechanisms of COMPOUND's utility as a bronchodilator/prophylactic drug as taught by the present invention.

As will be appreciated by those versed in the art, utility of COMPOUND in prophylactic, as opposed to symptomatic treatment of inflammatory airways disease is a property of very few known drug substances, principally of the two asthma prophylactic drugs ketotifen and SCG, neither of which, unlike COMPOUND, has the concomitant benefit of any symptomatic, e.g. bronchodilator, efficacy. By combining the benefits of both symptomatic (bronchodilator) and prophylactic utility as taught by the present invention, COMPOUND thus provides very significant and far reaching benefit over known or proposed drug therapy, e.g. over ketotifen or SCG.

When employed, e.g. as hereinafter described for the treatment of asthma, COMPOUND provides, as a bronchodilator, immediate or one-off symptomatic benefit by aborting or restricting asthma attack. On continued administration COMPOUND provides, as a prophylactic drug, the benefit of advanced protection against recurrence of asthma attack, or of control, restriction or reversal of basal causes of asthma and asthma attack e.g. as revealed by reduced intensity of airways hyperreactivity with continuing administration.

In accordance with the foregoing the present invention provides:

I. A method for the treatment of obstructive or inflammatory airways disease in a subject in need thereof, which method comprises administering to said subject an effective amount of COMPOUND; for example

Ia. A method of effecting bronchodilatation in a subject in need thereof (for example a subject exhibiting obstructive or inflammatory airways disease or airways obstruction, including chronic or acute obstruction, for example as occurring in the symptomatology of diseases, disorders or conditions as hereinafter set forth), which method comprises administering to said subject a bronchodilatorily effective amount of COMPOUND; or

Ib. A method for the prophylactic treatment of obstructive or, more particularly, inflammatory airways disease (e.g. for advance protective treatment against acute airways obstruction, for example bronchospasm, e.g. as occurring in the symptomatology of diseases, disorders or conditions as hereinafter set forth) in a subject in need thereof, which method comprises administering to said subject a prophylactically effective amount of COMPOUND (for example administering COMPOUND over a prolonged period of time at prophylactically effective dosage rate).

In the alternative the present invention provides:

II. COMPOUND for use in the treatment of obstructive or inflammatory airways disease, e.g. for use in a method as defined under I above, or

III. the use of COMPOUND in the preparation of a pharmaceutical composition for use in the treatment of chronic obstructive or inflammatory airways disease, e.g. for use in a method as defined under I above; for example

IIa. COMPOUND for use as a bronchodilator -, or

IIIa. the use of COMPOUND in the preparation of a pharmaceutical composition for use as a bronchodilator -,

- e.g. for use in a method as defined under Ia above; or

IIb. COMPOUND for use as a prophylactic drug for the treatment of inflammatory airways disease -, or

IIIb. the use of COMPOUND in the preparation of a pharmaceutical composition for use as a prophylactic drug for treatment of inflammatory airways disease -,

- e.g. for use in a method as defined under Ib above.

Having regard to the inherent activity of COMPOUND in suppressing eosinophil accumulation as recited under (d) above, and as hereinafter discussed in greater detail, the present invention also provides:

Ic. A method for the suppression of eosinophil accumulation and/or activation, e.g. for treating disease characterised by, or having an aetiology comprising, morbid eosinophil accumulation and/or activation, in a subject in need of such treatment which method comprises administering to said subject an effective amount of COMPOUND;

or, in the alternative:

IIc. COMPOUND for use as a suppressor of eosinophil accumulation -, or

IIIc. the use of COMPOUND in the preparation of a pharmaceutical composition for use in the suppression of eosinophil accumulation -,

- e.g. for use in a method as defined under Ic above.

COMPOUND exists in both (+)- and (-)-enantiomeric form, i.e. dependent on the absolute configuration across the carbon atoms 4a and 10b (the configuration being in either event cis). Individual (+)- and (-)-antipodes may be obtained by conventional techniques, e.g. by separation of the (±)-racemate, proceeding via separation of an appropriate (±)-racemic intermediate or by the use of appropriate optically active starting materials. Preferred methods for the preparation of individual (+)- and (-)-antipodes are described in the preparative example below.

References to COMPOUND, including references to COMPOUND by full chemical name, throughout the present specification and claims are to be understood as embracing both the individual (+)- and (-)-antipodes as well as mixtures thereof, e.g. (±)-racemic mixtures, unless otherwise specified. Thus "(±)-COMPOUND" designates the racemate specifically.

In so far as utility in accordance with the present invention is believed to be predominantly attributable to the (-)-antipode, use of the (-)-antipode or mixtures, e.g. racemic mixtures,

comprising the (-)-antipode is preferred. The (-)-antipode, whether in pure or substantially pure form or in admixture, e.g. racemic admixture, with the (+)-antipode is designated in the present specification and claims as "(-)-COMPOUND" or (-)-cis-6-(4-acetanilido)-8,9-dimethoxy-2-methyl-1,2,3,4,4a,10b-hexahydrobenzo[c][1,6]naphthridin. Whenever a specific degree of purity is intended this is particularised. Where mixtures of the (+)- and (-)-antipode are employed in accordance with the present invention, these will suitably be racemic mixtures, or mixtures comprising at least 40 %, preferably at least 50 % (-)-COMPOUND. (-)-COMPOUND in pure or substantially pure form may also be employed.

COMPOUND exists in both free and acid addition salt form. Pharmaceutically acceptable acid addition salts of COMPOUND, for example the dihydrochloride and bis-hydrogen maleate, exhibit an equivalent order of activity and degree of tolerability as the free compound. References to COMPOUND, including references to COMPOUND by full chemical name, throughout the present specification and claims are to be understood as embracing COMPOUND in both free and pharmaceutically acceptable acid addition salt form unless otherwise specified.

The words "treatment" and "treating" as used throughout the present specification and claims are to be understood as including prophylactic as well as symptomatic modes unless otherwise specified.

As will be appreciated "obstructive airways disease", "inflammatory airways disease" and "disease characterised by, or having aetiology comprising morbid eosinophil accumulation"

do not constitute mutually exclusive groups. Asthma for example is a disease commonly characterised by airways obstruction, which can be both acute, e.g. as in the course of asthma exacerbation or attack, or chronic, e.g. as in the case of "wheezy infants" (see below) or other adult asthmatics liable to persistant dyspnea. It is also the classic example of a disease characterised by airways inflammation, as well as morbid, pulmonary eosinophil infiltration, in particular evident during exacerbation or attack. And this commonly holds true for subjects exhibiting various forms of asthma, including extrinsic asthmas, e.g. allergic atopic asthmas, as well as intrinsic asthmas, i.e. for which there is no identifiable external cause. The inherent and essential unity of the present invention will thus be apparent.

The method of the present invention as defined under I or Ia to Ic above, especially as defined under Ia and Ib above, is in particular applicable to the treatment of asthma of whatever type or genesis. It is applicable to both intrinsic and, especially, extrinsic asthma. It is especially applicable to the treatment of allergic asthma, whether atopic, (i.e. IgE-mediated) or non-atopic, as well as e.g. bronchitic asthma, thymic asthma, excercise induced asthma, occupational asthma, asthma induced following bacterial infection and other non-allergic asthmas. Treatment of asthma is also to be understood as embracing treatment of subjects, e.g. of less than 4 or 5 years of age, exhibiting wheezing symptoms, in particular at night, and diagnosed or diagnosable as "wheezy infants", an established patient category of major medical concern and now more correctly identified as incipient or early-phase asthmatics. (For convenience of definition this particular asthmatic condition is referred to hereinafter and in the accompanying claims as "wheezy-infant syndrome").

In a series of particular embodiments the present invention thus provides for treatment of asthma, in particular allergic asthma (for example allergic atopic asthma), exercise induced asthma and wheezy-infant syndrome, including symptomatic treatment of asthma (e.g. bronchodilator treatment of asthma exacerbation or attack) as well as prophylactic treatment of asthma (e.g. prophylactic treatment of asthma exacerbation or attack), entailing use of, or administration of, COMPOUND.

The method of the present invention as defined under I or Ia to Ic above, especially as defined under Ia and Ib above, is also applicable to the treatment of pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and, in particular, byssinosis.

In a further series of particular embodiments the present invention thus also provides for the treatment of pneumoconiosis, in particular byssinosis, including symptomatic treatment of airways obstruction (e.g. bronchodilator treatment of acute or chronic airways obstruction, e.g. dyspnea or bronchospasm) attributable thereto, as well as prophylactic treatment of airways obstruction (e.g. advance protective treatment of acute airways obstruction, e.g. bronchospasm) attributable thereto, comprising use or administration of compound.

The method of the present invention as defined under I or Ia to Ic above, for example as defined under Ic above, is also applicable to the treatment of morbid eosinophil accumulation

and/or activation including hypereosinophilia and the eosinophil-related disorders.

Hypereosinophilia is a distinct condition or status of varied aetiology characterised by chronic, morbid eosinophil presence in the body tissues generally. The eosinophil-related disorders comprise a distinct and extensively documented indication group, commonly occurring concomitant to another, primary disease or condition. [For more detailed discussion see e.g.: Schatz et al., Medical Clinics of North America, 65, (5), 1055-1071 (1981) and Ottesen et al., "Allergy, Principles and Practice", Eds.E. Middleton, C. Reed and S. Ellis, 584-632, (1987)]. The group includes eosinophil-related disorders of the airways (involving morbid eosinophilic infiltration of pulmonary tissues) as well as of other organs and tissues including, for example, the skin, eye, nasal passages and the gastro-intestinal and urinary tracts.

Eosinopil-related disorders to which the present invention is applicable include those concomitant to atopy or atopic reactions in general (e.g. atopic conditions such as rhinitis, conjunctivitis etc... as set forth below) as well as non-atopic eosinophil-related disorders.

Disorders of the airways to which the present invention is applicable include hypereosinophilia as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome) as well as eosinophil-related disorders affecting the airways occasioned by drug-reaction.

Other eosinophil-related disorders to which the present invention is applicable include eosinophilia consequential or concomitant to eosinophilic gastroenteritis, Heiner's syndrome, atopic dermatitis, urticaria or angioderma (allergic, recurrent or prolonged), ichthyosis, exfoliative dermatitis or pityriasis rubra, urticaria pigmentosa or mastocytoma, toxic epidermal necrolysis (drug related), dermatitis herpetiformis, allergic rhinitis, hyperplastic sinusitis, interstitial nephritis (drug related), interstitial cystitis, choleostatic hepatotoxicity (drug related), allergic conjunctivitis, vernal conjunctivitis, eosinophilic fascitis, hypersensitivity angiitis, serous myocarditis or endomyocardial fibrosis, Wiscott-Aldrich syndrome, selective IgA deficiency with atopy, eosinophilic leukemia and eosinophilic granuloma.

As will be appreciated, the present invention is directed primarily to the treatment of hypereosinophilia or eosinophil-related disorders as such. Where, however, eosinophil-related disorders are concomitant to atopy, for example to any of the atopic diseases or conditions specifically recited above, including atopic or allergic forms of dermatitis, urticaria, angioderma, rhinitis, conjunctivitis and gastro-intestinal allergies, the present invention is equally applicable to the treatment of eosinophil-related disorders as an integral or basal component thereof. The present invention thus also provides means

for the treatment (e.g. symptomatic or prophylactic treatment) of atopy, including each of the said recited atopic diseases or conditions, as such. In treating eosinophil-related disorders concomitant to non-atopic diseases or conditions on the other hand, COMPOUND will more commonly be administered together with other medication for treatment of the disease or condition with which eosinophilia is associated. Thus in the treatment of eosinophilia consequential to parasitic infection, compound will generally be administered in conjunction with other, anti-parasitic drug therapy.

Where COMPOUND is employed in accordance with the method of the invention for the treatment of eosinophil-related disorders of the airways, e.g. for the treatment of hypereosinophilia as it affects the lungs or for the treatment of pulmonary eosinophilia associated with eosinophilic pneumonia, and the disorder is accompanied by symptoms of airways obstruction, for example dyspnea, COMPOUND may be employed either as symptomatic or prophylactic therapy, e.g. either to alleviate or abort, or to provide advance protection against recurrence of, obstruction.

More commonly however COMPOUND will be employed symptomatically, e.g. as a means for the treatment of hypereosinophilia or eosinophil-related disorder, i.e. in accordance with methods defined under Ic above.

In a further series of particular embodiments the present invention thus also provides:

i) for the treatment of hypereosinophilia and of eosinophil-related disorders, including treatment in accordance with the methods defined under Ic above, including, in the case of eosinophil-related disorders of the airways associated with airways obstruction, symptomatic treatment of airways obstruction (e.g. bronchodilator treatment of acute or chronic airways obstruction, e.g. dyspnea or bronchospasm) and prophylactic treatment of airways obstruction (e.g. advance protective treatment of acute airways obstruction, e.g. bronchospasm) attributable thereto, comprising use or administration of COMPOUND; as well as

ii) for the treatment of atopy, for example for the treatment of any of the atopic diseases or conditions causal to or associated with eosinophil-related disorder as hereinbefore set forth, comprising use or administration of COMPOUND.

The method of the present invention as defined under I or, especially, Ia above, is also applicable to the treatment of bronchitis or, more especially, for the treatment of chronic or acute airways obstruction, for example, dyspnea, associated therewith. In this respect the present invention is applicable to the treatment of bronchitis of whatever type or genesis, including, for example, acute bronchitis, arachidic bronchitis, catarrhal bronchitis, chronic bronchitis, croupous bronchitis, phthinoid bronchitis and so forth.

In a further series of particular embodiments the present invention accordingly provides for the treatment of bronchitis or, more especially, the symptomatic treatment of airways obstruction (e.g. bronchodilator treatment of acute or chronic

airways obstruction, e.g.dyspnea) attributable thereto, comprising use or administration of COMPOUND.

The following examples are illustrative of the present invention and means for putting the invention into practice.

PREPARATIVE EXAMPLE

COMPOUND may be prepared in accordance with the general procedures described in US Patent No. 3,899,494, e.g. proceeding in accordance with the sequence of the following FLOW-CHART. Where individual (+)- or (-)-antipodes of COMPOUND are required, isomer separation is preferably performed on the intermediate (2) (see FLOW-CHART).

FLOW-CHART

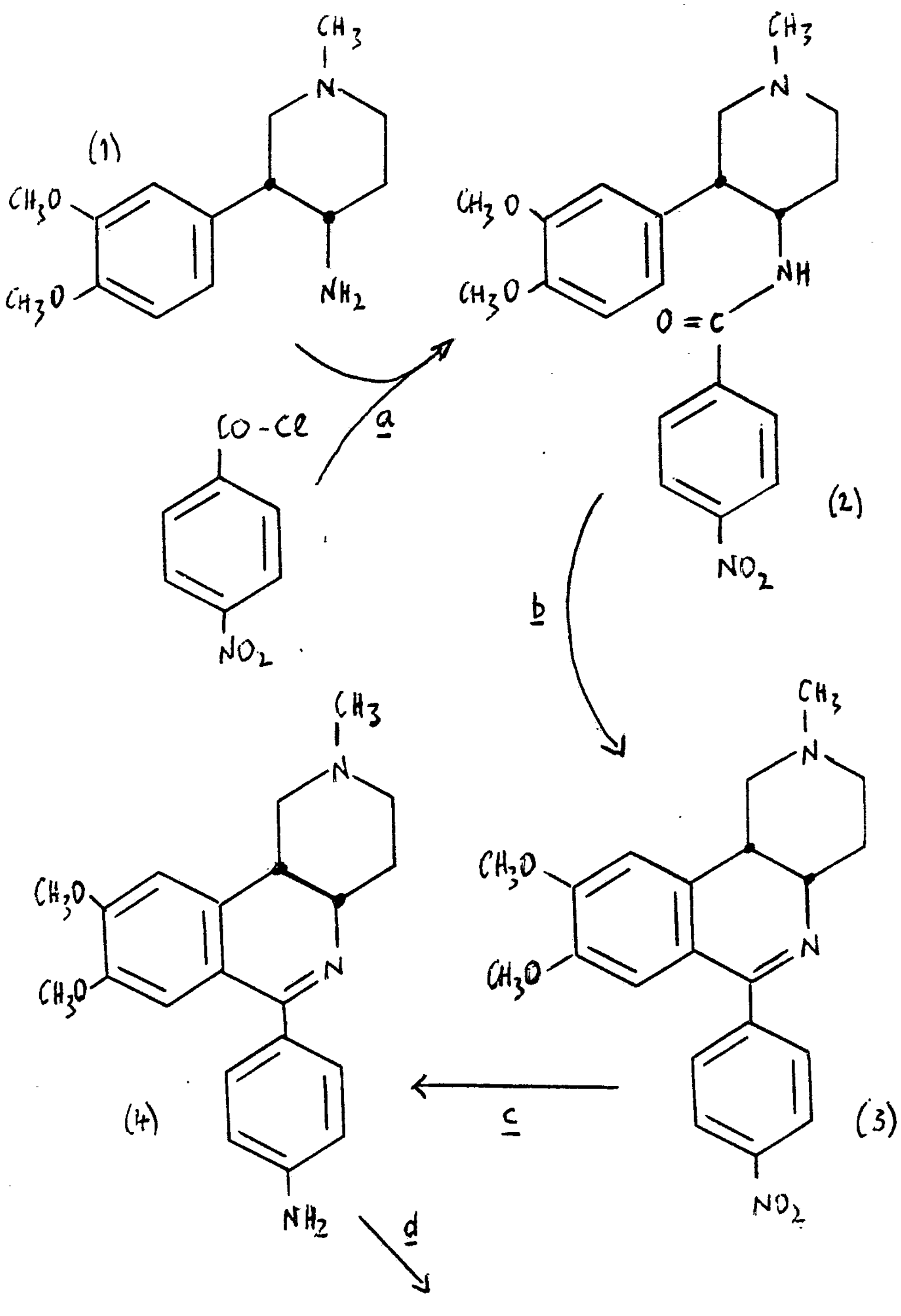


(1)

(2)

(3)

(4)

COMPOUND

1a. Preparation of the (+)-antipode of intermediate (2):

12 g (2) [obtained from (1) in accordance with the procedures described in USP 3,899,494] are dissolved hot in 200 ml ethanol and 4.5 g L-tartaric acid are added. Crystalised L-tartrate is filtered off after cooling and dried: m.p. = 170 - 172°C. Raw [(+)-(2)] is freed from the salt with NaOH/$CH_2Cl_2$: $[\alpha]_{578}$ = + 64° (c = 1.0 in $CHCl_3$). The obtained [(+)-(2)] is again treated with 2.14 g L-tartaric acid and freed with NaOH/$CH_2Cl_2$ as previously described: $[\alpha]_{578}$ = + 73°. Following re-crystallisation 2x from ethyl acetate, pure [(+)-(2)] is obtained: $[\alpha]_{578}$ = +79° (c = 1.0 in $CHCl_3$).

1b. Preparation of the (-)-antipode of intermediate (2):

The mother-liquor remaining from the first crystalisation described in 1a above is shaken with NaOH/$CH_2Cl_2$ to yield raw [(-)-(2)]: $[\alpha]_{578}$ = -71.5° (c = 1.0 in $CHCl_3$). The raw product is treated with 2.05 g D-tartaric acid, to yield (D)-tartrate and the crystallised product treated with NaOH/$CH_2Cl_2$ to yield [(-)-(2)]: $[\alpha]_{578}$ = -746°. Following re-crystallisation from ethyl acetate, pure [(-)-(2)] is obtained: $[\alpha]_{578}$ = -79.5° (c = 1.0 in $CHCl_3$).

2a. Preparation of the (-)-antipode of intermediate (3): FLOW-CHART, step b:

15.4 g [(+)-(2)] are treated under reflux for 3 hrs. in 100 ml $POCl_3$ and 300 ml absolute toluene, whereupon an oily, later crystalline, residue develops. The residue is separated after cooling and tipped into ice water. The mixture is rendered alkaline with $K_2CO_3$ and then extracted with methylene chloride.

The residue is recrystallised from ethanol to yield [(-)-(3)]: $[\alpha]_{578}$ = -214.5° (c = 1.0 in $CHCl_3$): m.p. = 170 - 171°C.

2b. Preparation of the (+)-antipode of intermediate (3):

The title product is obtained analogously to 2a above starting from [(-)-(2)]:$[\alpha]_{578}$ = +221° (c = 1.0 in $CHCl_3$): m.p. = 172 - 173°.

3a. Preparation of the (+)-antipode of intermediate (4): FLOW-CHART, step c:

6.6 g Iron filings are added to 6.9 g [(-)-(3)] dissolved with heating in 40 ml ethanol, 5 ml conc. HCl and 40 ml $H_2O$. The whole is stirred vigorously for 1.5 hrs. with heating under reflux. The hot reaction mixture is filtered and concentrated. The aqueous phase is treated with $CH_2Cl_2$ and then NaOH and the iron oxide precipitate filtered off. After separation and drying of the organic phase and chromatographic purification, [(+)-(4)] is obtained: $[\alpha]_{578}$ = +206° (c = 1.0 in $CHCl_3$).

3b. Preparation of the (-)-antipode of intermediate (4):

The title product is obtained analogously to 3a above starting from [(+)-(3)]: $[\alpha]_{578}$ = -210° (c = 1.0 in $CHCl_3$).

4a. Preparation of (+)-COMPOUND in pure form: FLOW-CHART, step d:

3.7 g [(+)-(4)] are heated with 15 ml acetic acid anhydride and 15 ml pyridine for 2 hrs. at 60°C. The reaction mixture is evaporated to dryness under vacuum and the residue poured onto water and allowed to stand for 30 mins. After rendering alkaline

with $K_2CO_3$, the whole is extracted with $CH_2Cl_2$, dried and the organic phase evaporated off to yield (+)-COMPOUND in pure form: $[\alpha]_{578}$ = +165° (c = 1.0 in $CHCl_3$).

4b. Preparation of (-)-COMPOUND in pure form:

(-)-COMPOUND in pure form is obtained analogously to 4a starting from [(-)-(4)]: $[\alpha]_{578}$ = -183° (c = 1.0 in $CHCl_3$).

(±)-COMPOUND may be prepared analogously to the above procedures, but with omission of antipodé separation procedures 1a and 1b.

Utility of COMPOUND in accordance with the present invention can be demonstrated in standard laboratory test models as well as in clinical trials, e.g. performed as follows:

EXAMPLE 1: BRONCHODILATOR ACTIVITY

1a. Bronchospasmolytic Activity in vitro

1a$^1$. Isolated guinea-pig trachea:

The trachea is excised from freshly sacrificed guinea-pigs and transected in the transverse plane to give rings of tissue of ca. 2 mm. Individual rings are mounted vertically on stainless steel supports, one of which is fixed at the base of an organ bath, the other being attached to a tension transducer. The rings are bathed in modified Tyrode solution at 37°C and gassed with $O_2/CO_2$ (95:5, v/v). Rings prepared in this manner, preloaded with 1 g, generate spontaneous tone and, after a period of

equilibration, relax on addition of spasmolytic drugs. Tension can be enhanced by addition of carbachol ($10^{-6}$M) or histamine ($10^{-4}$M). To ascertain spasmolytic activity, test substances are dissolved in physiological saline and added in increasing quantities to the organ bath at 5 min. intervals to provide a cumulative concentration-effect curve.

In the above test model COMPOUND produces concentration-related relaxation of guinea-pig tracheal ring preparations, irrespective of the contractile agency. Thus in one series of tests, established $IC_{50}$ values for (±) COMPOUND, for inhibition of spontaneous tone and contraction due to carbachol and histamine are $3.2 \times 10^{-6}$M, $5.8 \times 10^{-6}$M and $1.7 \times 10^{-6}$M respectively. For comparison, Aminophylline established $IC_{50}$ values using such tissue preparations are of the order of from $2.7 \times 10^{-4}$ to $4.7 \times 10^{-5}$M.

$IC_{50}$ values that are independent of the spasmogen employed, differentiate COMPOUND from parasympatholytic drug substances which exhibit preferential inhibition of responses to carbachol.

Furthermore employing the above test model, the relaxant action of COMPOUND [e.g. (±)-COMPOUND] is found to be undiminished in the presence of propanolol (1 μM) and is not influenced by the use of tracheal rings prepared from animals which have been previously desensitised to $\beta_2$-agonist therapy by prolonged exposure to high-dose isoprenaline _in vivo._ The relaxant action of COMPOUND is thus also distinguished from that of $\beta_2$-agonist drug substances.

1a2. Isolated Human Bronchus:

Rings of bronchus (ca. 2mm depth) are dissected from samples of human lung, resected from patients with lung carcinoma but grossly free of disease. Activity is determined employing the methodology of example 1a1.

In the above test model COMPOUND produces contraction-related relaxation of human bronchus ring preparations, irrespective of the contractile agency. Thus in one series of tests, established $IC_{50}$ values for (±)-COMPOUND for inhibition of spontaneous tone and for contraction by carbachol and histamine are of the order of from $4.7 \times 10^{-6}$ to $8.7 \times 10^{-7}$M. For comparison, aminophylline gives $IC_{50}$ values of the order of from $1.4 \times 10^{-4}$ M and $8 \times 10^{-6}$ M.

## 1b. Bronchodilator Activity in vivo

1b1 Inhibition of bronchospasm:

Guinea pigs are anaesthetised with pentobarbital (30 mg/kg i.p.) and phenobarbital (100 mg/kg i.p.) and ventilated via a tracheal cannula (10 ml/kg, 1Hz). Ventilation is monitored either by a pressure transducer measuring air-flow (Konzett-Rossler method), or by a Fleisch flow transducer in line with the inspiratory circuit. When making measurements of flow, coincident pressure changes in the thorax are monitored directly via an intrathoracic trochar, permitting display of differential pressure relative to the trachea. From this information resistance and compliance are calculated at each inspiration. Test substance is, administered via an indwelling cannula within a jugular vein. Blood pressure

is monitored at the carotid artery. Test substance is administered immediately before injection of a bronchoconstrictor agent - PAF (56 ng/kg), acetylcholine (32 µg/kg), histamine(10 µg/kg), serotonin (10 µg/kg), substance P (10 µg/kg) or leukotriene $C_4$ (5 µg/kg).

In the Konzett-Rossler preparation, COMPOUND [in the case of (±)-COMPOUND, at dosages of the order of 1 mg/kg] strongly inhibits (pre-empts) constrictor response to each of the above recited bronchoconstrictor agents.

$1b^2$ Reversal of bronchospasm:

Guinea-pigs are prepared for measurement of resistance and compliance as under $1b^1$ above. Intravenous injection of bombesin (ca. 500 ng/kg) as a bolus, induces increased airways resistance which is sustained over a period of several minutes. Capacity of test-substance to reverse response when administered i.v. at the height of bombesin-induced bronchospasm serves as a measure of efficacy in reversing established bronchospasm.

In the above test model, COMPOUND is found to effect dose related abrogation of bronchospasm, determined $ID_{50}$ values for (±)COMPOUND in one series of tests being of the order of 0.08 - 0.48 mg/kg.

$1b^3$ Inhibition of bronchoconstriction following pulmonary administration.

Conscious guinea-pigs are subjected to inhalation of COMPOUND or placebo (vehicle) 10 mins. prior to explosure to a 0.3% mist of acetylcholine. COMPOUND is administered as a mist generated

from COMPOUND in aqueous solution at concentrations of from 1 mg/ml to 0.001 mg/ml, using a jet nebuliser. Prolongation of time prior to collapse in treated groups as compared with placebo groups is taken as a measure of bronchodilator efficacy.

In the above test model, detecable protection against bronchospasm is evident employing COMPOUND, in the case of (±)-COMPOUND on introduction into the exposure chamber in amounts of from 2µg upwards.

EXAMPLE 2: SUPPRESSION OF AIRWAYS HYPERREACTIVITY

2a Sensitised Ainimals

Guinea pigs are anaesthetised and airways resistance and compliance recorded as described under example $1b^1$ above. Intravenous injection of histamine (1 - 1.8 µg/kg) is used to define airways sensitivity. Platelets are isolated and labelled with $^{111}$indium prior to intravenous infusion. Platelet accumulation is monitored by external crystal detectors [c.f. Morley et al. Trends in Pharmacological science 5,258-261 (1984)], using an automated isotope monitor system. Allergic reaction is initiated by i.v. injection of preformed immune complexes (bovine γ-globulin/anti-bovine γ-globulin), using a dose that is scarcely sufficient to induce bronchospasm at the first injection. By repeating the dose at regular (10 min.) intervals, bronchospasm of progressively increasing amplitude is evidenced. This effect is paralleled by an incremental retention of platelets within the airway vasculature. Following the last dose of immune complexes, the dose-effect relationship to histamine is re-defined. In animals so treated, induction of airways hyperreactivity is consistently observed.

On advance administration of COMPOUND [in the case of (±)-COMPOUND, at dosages of from ca. 1 mg/kg i.v. upwards] suppression of induced airways resistance is observed, as compared with untreated controls. The dose-effect relationship to histamine is reduced by comparision with control animals and at higher dosages can be fully abolished, such that the level of airway sensitivity becomes lower than that observed prior to treatment, even though no bronchodilator activity for COMPOUND may be detectable at this time. No inhibition of platelet accumulation is evident. Inhibition of airways hyperreactivity by COMPOUND is found to be dose dependent, with an effective dose for (±)-COMPOUND of 0.35 mg/kg being determined in one test series.

2b PAF-Treated Animals

Guinea-pigs are anaesthetised and prepared for recording of lung function as described under example $1b^1$ above. Intravenous injection of low dose bombesin (240 ng/kg) establishes airways sensitivity to spasmogens. Following infusion of PAF over 1hr. (total dose = 600 ng/kg), repeated injection of test dose bombesin (240 ng/kg) reveals development of airways hyperreactivity, which can conveniently be expressed as the paired difference between the response amplitude before and after PAF exposure. Induction of airways hyperreactivity by PAF is a platelet-dependent phenomenon and development of airways hyperreactivity in this model in paralleled by observed accumulation of platelets within the thorax.

On advance administration of COMPOUND [in the case of (±)-COMPOUND at dosages of from ca. 0.1 mg/kg i.v. upwards] suppression of airways hyperreactivity induction is observed. The inhibitory effect of COMPOUND is dose dependent, an established $ID_{50}$ for (±)-COMPOUND in one series of tests being of the order of 1 mg/kg i.v. In addition, in animals receiving COMPOUND bolus, followed by COMPOUND infused i.v. [in the case of (±)-COMPOUND, following e.g. 1 mg/kg bolus and infusion at 1 mg/kg/hr] thoracic platelet accumulation in response to PAF is also inhibited.

EXAMPLE 3: INFLUENCE ON EOSINOPHIL ACCUMULATION

Effect of test-substance is conveniently determined by measurement of influence on PAF-induced eosinophil accumulation in the guinea-pig peritoneal cavity in vivo. In the guinea-pig, there is a substantial (up to 40%) resident population of eosinophils in the peritoneal cavity and eosinophil accumulation in the peritoneal cavity relative to control animals ca. 24 - 48 hrs. following injection of PAF i.p. at dosages of ca. 10µg/kg.

To esablish eosinophil accumulation, test animals receive 10µg/kg PAF i.p., 2 days prior to sacrifice. Smears from the peritoneal cavity are prepared employing Leishman's Stain after fixation with 95% methanol. At least 500 white cells are differentiated for each estimation. Test substance is administered s.c. via a minipump for four days prior to sacrifice.

On administration of COMPOUND in the above test model [in the case of (±)-COMPOUND, at e.g. dosages of the order of 10 mg/kg/day] for four days prior to sacrifice and employing non-PAF-treated animals, decrease in resident eosinophil population is observed as compared with untreated controls.

On administration of COMPOUND in the above test model [in the case of (±)-COMPOUND, at e.g. dosages of the order of 2 mg/kg/day] for four days prior to sacrifice and employing animals receiving PAF as described above, inhibition of eosinophil accumulation is observed as compared with controls receiving PAF but not COMPOUND.

CLINICAL TRIAL

A. Bronchodilator efficacy of COMPOUND compared with placebo and/or Theophylline and with Salbutamol

Subjects chosen for the trial are patients exhibiting bronchial asthma (suitably with perennial symptoms) and identified as requiring $\beta_2$-agonist therapy, and/or other bronchodilator anti-asthmatic drug therapy.

Criteria for inclusion are based on medical history and on initial examination. Subjects selected are of either sex, aged between 18 and 60 years, and identified as having a history of bronchial asthma and positive response to inhalation of $\beta_2$-agonist therapy (e.g. Fenoterol or Salbutamol) or other bronchodilator therapy, e.g. with FEV-1 and/or PEFR increasing by 20% as documented 1 week before commencement of the trial. Baseline of FEV-1 and/or PEFR in each subject entered for the trial should be at least 20% below the predicted value, preferably also at the time of trial. The following exclusion criteria are applied:

- Severe chronic obstructive pulmonary disease, emphysema, active and progressive infection, status asthmaticus during the last 12 months.
- Any serious or chronic illness of the gastrointestinal system, liver or kidneys which may influence absorption, metabolism and excretion of the drugs.
- Diabetes mellitus.
- Any condition which might impair the ability of the physician to evaluate the progress of the patient.

Subjects are advised not to change normal life during the course of the trial.

Medication employed for the trial comprises:

A. (±)-COMPOUND (in the form of its bis-hydrogen maleate salt), in dry substance form. For administration the active ingreded is dissolved in pharmaceutical grade water, 30 - 60 mins. before ingestion: at a concentration of 1 g/100 ml, for administration at a dosage of 300 mg (30 ml), 200 mg (20 ml) or 100 mg (10 ml); at a concentration of 0.5 g/100 ml, for administration at a dosage of 50 mg (10 ml); or at a concentration of 0.25 g/100 ml for administration at a dosage of 25 mg (10 ml).

B. Theophylline solution at a dosage of 150 mg.

C. Placebo - aqueous solution only, no active ingredient.

D. Salbutamol, administered by inhalation in 3 puffs comprising 0.1 mg active ingredient/puff.

The trial is performed for each subject on 2, 3 or more separate days, each trial day being separated by a wash-out period of 3 to 7 days. On each trial day each subject receives medication A, B

or C in the morning, medication being assigned in single-blind, randomised, cross-over fashion from trial day to trial day. Seven hours later all subjects receive medication D (open) on each trial day. The night prior to each trial, no alcoholic beverages are permitted. Opening medication (A, B or C) is taken 60 mins. after breakfast (no coffee).

Lung-function (Lufu) assessment is performed before and repeated during the course of and post-trial (i.e. subsequent to medication D). No $\beta_2$-agonist medication is permitted less than 6 hours, preferably less than 12 hours, and no methyl-xanthine therapy is permitted less than 24 hours, preceeding trial commencement.

The following parameters are recorded: General medical history, general condition, skin, locomotor system, eyes, nose, throat, cardiovascular system, thorax, blood pressure and pulse rate (after 5 mins. rest), body weight and height.

The following Lufu tests are performed:

a) FVC; FEV 0.5; FEV 1.0; FEV 3.0;
b) FEV 0.5/FVC; FEV 1/FVC; FEV 3/FVC;
c) PEF; FEF 25-75%; FEF 75-85%; FEF 25%; FEF 50%; FEF 75%; FEF 0.2 - 1.2
d) Flow expiratory curve and flow inspiratory curve.

Tolerance of medication and possible occurrence of side-effects are also reported.

In the above clinical trial COMPOUND [(±)-COMPOUND in bis-hydrogen maleate form] is found to provide marked improvement in lung-function on administration at above indicated dosage rates as compared with placebo. In particular at higher dosage rates, e.g. of 50 mg or 100 mg and more, results obtained approach or are at least comparable with results obtained employing therapy B or D. In all instances COMPOUND is found to be well tolerated both in terms of reported occurrence of side-effects and reported or observed side-reactions.

As an alternative, the above trial may also be performed in analogous fashion employing COMPOUND [e.g. (±)-COMPOUND in bis-hydrogen maleate form] administered by inhalation, e.g. as an aqueous spray, e.g. at dosages of from 1 to 50 mg, suitably from 5 to 25 mg, for example at dosages of 5, 10, 15, or 20 mg.

The trial may also be extended to embrace multiple dosaging with medication A, e.g. administered 2 or 3x daily over a period of days, with serial lung function monitoring as described above.

B. Prophylactic efficacy of COMPOUND compared with placebo

Prophylactic efficacy of COMPOUND [e.g. (±)-COMPOUND in bis-hydrogen maleate form] may be determined in clinical trials of classic design, e.g. involving subjects exhibiting allergic, atopic bronchoconstrictor response, by administration of test medication or placebo 1 to 2 hours prior to allergen challenge, or by administration of test medication at regular dosage rates (e.g. 2 or 3x daily) or placebo over a period of 1 to 7 days prior to allergen challenge.

Suitable individual dosages, e.g. for (±)-COMPOUND as the bis-hydrogen maleate, in such clinical trials comprise e.g. ca. 1 to 25 mg administered by inhalation (for single administration or administration 2 to 3 daily) or ca. 25 to 100 mg orally (for single administration or administration 2 to 3x daily) up to ca. 200 or 300 mg orally (for single administration).

Both acute (0 to 1 hour) and long-term (up to 12 hour) change in lung function is determined post-medication by measurement of parameters (FVC, FEV 1 etc...) as set forth for clinical trial A above.

In addition to monitoring of lung function, blood eosinophil levels are also monitored and the incidence of activated eosinophils and eosinophil secretory proteins is determined. In individual studies, subjects are also subjected to lung lavage and the presence of eosinophils and platelets and their respective secretory products determined before and after exposure to allergen.

In clinical trials designed in accordance with the above principles, COMPOUND [e.g. (±)-COMPOUND in bis-hydrogen maleate salt form] provides advance protection against bronchorestrictor response to allergen, as well as reduction of eosinophilic response, compared with control subjects receiving placebo only, on administration at dosages indicated.

C. Prophylactic/bronchodilator efficacy in asthma

Prophylactic efficacy of COMPOUND [e.g. (±)-COMPOUND in bis-hydrogen maleate form], may also be demonstrated in clinical

trials involving administration to groups of asthmatic subjects on a regular daily basis over periods from at least 2, preferably 3 months, up to 6 months and more. Initial dosaging is of the order of from 1 to 100 mg, e.g. from 5 to 50 mg per day, administered once or in divided dosages 2 or 3x daily, for administration by inhalation and of the order of from 25 to 300 mg per day administered once or in divided dosages 2 or 3x daily, for oral administration, with subsequent reduction in appropriate cases to a maintainance dosage of 1 to 50 mg, e.g. from 5 to 25 mg, per day for inhalation or from 25 to 100 or 200 mg per day orally. Administration is suitably effected in divided dosages 2 or 3x daily. During the course of the trial subjects are monitored continually and all relevant parameters recorded including lung function, alterations in the incidence of activated eosinophils or the release of eosinophil products into the blood or bronchial fluids and/or changes in the function of platelets *ex vivo* and, in particular, frequency and severity of asthma exacerbation or attack. Additional bronchodilator dosaging may be provided on the occasion of any such attack.

Subjects receiving therapy in accordance with the invention show marked reduction in frequency of asthma exacerbation or attack, tending, with duration of trial, to lead to complete freedom from attack as compared with subjects receiving alternative, non-prophylactic therapy. Where participating subjects are initially dependant on concomitant, bronchodilator, e.g. methyl-xanthine or $\beta_2$-agonist drug therapy, increasingly reduced need for concomitant therapy is also observed. Results employing COMPOUND at exacerbation or attack indicate symptomatic efficacy, e.g. in abrogating or treating attack on occurrence.

D. Efficacy in pneumoconiosis

The trial is performed analogously to C above but with groups of subjects having a history of pneumoconiosis, e.g. characterised by events of restricted lung function, for example including difficulty in breathing, wheezing, dyspnea or occasional bronchoconstrictor attack and a record of pneumoconiosis-related work absenteeism. Suitable subjects include cotton-mill workers exhibiting severe byssionosis.

COMPOUND [e.g. (±)-COMPOUND in bis-hydrogen maleate form] is administered daily at dosage rates as set forth under C above, over periods in excess of 2, preferably in excess of 3 months.

During the course of the trial, subjects are monitored continually and all relevant parameters are recorded, including basal lung-function, frequency of attack with impairment of lung-function, requirement for other medication and frequency of absenteeism attributable to pneumoconiosis.

Subjects receiving therapy in accordance with the invention exhibit overall improvement in basal lung-function during the course of the trial accompanied by reduced frequency of attack and absenteeism as well, in cases where frequent use of alternative medication is recorded at trial entry, reduced need for concomitant medication.

E. Efficacy in hypereosinophilia and eosinophil-related disorders

The trial is performed analogously to C above but with groups of subjects exhibiting elevated eosinophil presence in the blood

attributable to eosinophil-related disorder, e.g. accompanying Loffeler's syndrome, eosinophilic pneumonia, eosinophilic fascitis, or attributable to hypereosinophilia.

COMPOUND [e.g. (±)-COMPOUND in bis-hydrogen maleate form] is administered orally at a daily dosage rate of from 25 to 800 mg, suitably 100, 200, 300, 500 or 800 mg. The trial is continued for a period of up to 2 to 3 months. Blood samples are collected regularly during the course of the trial and assayed for blood-eosinophil count, incidence of activated eosinophils and levels of eosinophil secretory proteins.

Subjects receiving therapy in accordance with the invention are found to exhibit decreased blood-eosinophil count during the course of treatment accompanied by decreased incidence of activited eosinophils and eosinophil secretory protein levels. Recorded symptomatology attributable to condition is also found to undergo reversal during treatment.

COMPOUND is well tolerated at dosages required for use in accordance with the invention. Thus toxicological studies in animals indicate $ED_{50}$ values for (±)-COMPOUND in the mouse, rat and rabbit of 163, 224 and 108 mg/kg respectively on i.v. administration and of 2433, 1091 and 423 mg/kg respectively on oral administration. 30-week oral administration of (±)-COMPOUND to rats and dogs indicate no toxicity at dosages up to 272 mg/kg/day in rats and up to 10 mg/kg/day in dogs. (±)-COMPOUND is found to have no embryolethal or teratogenic actions in the rat at dosages up to 300 mg/kg and in the rabbit at dosages up to 30 mg/kg.

As regards tolerability and safety in humans, single oral dosages of (±)-COMPOUND up to 800 mg to healthy trial volunteers (27 subjects) are found to produce no occurrence of side effects or relevant changes in laboratory parameters. A dose of 600 mg (±)-COMPOUND, once daily for 3 days (6 subjects) is found to be well tolerated. In subjects exhibiting heart insufficiency, oral dosages of up to 600 mg (±)-COMPOUND are found to produce no significant side effects and to be well tolerated. Placebo controlled trials indicate a light increase in systolic blood-pressure after 60 mins., with no relevant change in heart frequency. Similarly intravenous dosages of up to 100 mg (±)-COMPOUND administered by perfusion over 10 mins. to subjects exhibiting heart insufficiency are well tolerated, the only side effects recorded being light fall in arterial pressure accompanied by an increase in heart frequency.

Dosages of COMPOUND employed in practicing the method of the invention will of course vary depending e.g. on the mode of administration (for example whether oral or by inhalation), the particular condition to be treated, the severity of the condition, the therapy desired (e.g. in the case of asthma, whether bronchodilatory and/or prophylactic) as well as the time course of therapy (e.g. whether therapy comprises initiating treatment or maintainance therapy).

In general however, for (±)-COMPOUND satisfactory results are obtained on administration to patients, e.g. exhibiting asthma, pneumoconiosis, hypereosinophilia or eosinophil-related disorder

(as well as atopy, e.g. atopic diseases and conditions, as such), at an oral dosage rate of from ca. 5 to 300 mg/day or, in case of eosinophil-related disorder (or atopy as such), up to 800 mg/day, e.g. of from 25 to 200 mg/day or, in the case of hypereosinophilia or eosinophil-related disorder (or atopy as such) up to 500 or 800 mg/day, administered once, in divided dosages 2 to 3x daily, or in sustained release form. Where prophylactic efficacy alone is desired, e.g. for maintainance therapy in the treatment of asthma, dosage rates may tend to be at the lower end of the above indicated range, e.g. of the order of from 5 to 100, e.g. 10, 25 or 50 mg/day. Where bronchodilator efficacy is required, e.g. for symptomatic treatment during the initiating phase of asthma therapy, dosages may tend to be at the upper end of the above indicated range, e.g. of the order of from 25 to 300, e.g. 50, 100, 200 or 300 mg/day.

Suitable unit dosage forms for oral administration of (±)-COMPOUND in practicing the method of the present invention thus comprises e.g.:

i) for use in the treatment of asthma, pneunoconiosis, hypereosinophilia or eosinophil-related disorder, as well as atopy as such, from ca. 1.5 to 300, for example 5,10,25,50, 100 or 200 mg (±)-COMPOUND, together with an inert pharmaceutically aceptable diluent or carrier therefor; or

ii) for use in the treatment of hypereosinophilia or eosinophil-related disorder, as well as atopy as such, up to 800 mg, for example 200, 400, or 500 mg (±)-COMPOUND, together with an inert pharmaceutically acceptable diluent or carrier therefor.

Where administration by inhalation is contemplated, e.g. for the treatment of asthma exacerbation or attack, dosages of lower order may be appropriate, e.g. of ca. 1 to 75 or 100 mg/day for example 5 to 50 mg/day (±)-COMPOUND, with administration 1 - 3x daily.

In so far as activity in accordance with the invention resides primarily in its (-)-antipode, where (-)-COMPOUND is employed in pure or substantially pure form in place of (±)-COMPOUND, appropriate dosages will be of the order of ca. 50% of those indicated above. Unit dosage forms comprising (-)-COMPOUND plus inert pharmaceutically acceptable diluent or carrier will thus appropriately contain ca. 50% less active ingredient.

Pharmaceutical compositions comprising COMPOUND may be prepared in conventional manner, e.g. by admixure with an appropriate pharmaceutically acceptable solid or liquid diluent or carrier.

Suitable oral formulations for use in accordance with the present invention may be prepared, e.g. as follows:

1. CAPSULE

| Ingredient | (mg) |
|---|---|
| (±)-COMPOUND (in bis-hydrogen maleate form) | 1.06, 5.3, 10.6, 26.5, 53, 106, 318 or 530 |
| lactose | 46.0 |
| corn starch | 18.0 |
| magnesium stearate | 6.0 |
| silicon dioxide colloidal | 2.0 |

The ingredients are admixed in conventional manner and filled into regular hard or soft gelatin capsules to provide unit dosage forms comprising the equivalent of 1, 5, 10, 25, 50, 100, 300 or 500 mg free (±)-COMPOUND respectively, and suitable for use in accordance with the method of the invention.

2. ORAL LIQUID SUSPENSION

| ingredient | (mg) |
|---|---|
| (±)-COMPOUND (in bis-hydrogen maleate form) | 53.0 or 159 |
| sodium carboxy methylcellulose U.S.P. | 12.5 |
| magnesium aluminium silicate | 47.5 |
| flavour | q.s. |
| colour | q.s. |
| methyl paraben, U.S.P. | 4.5 |

Ingredient (cont.)

| | |
|---|---|
| propyl paraben, U.S.P. | 1.5 |
| polysorbate 80 (e.g. Tween 80), U.S.P. | 5 |
| sorbitol solution, 70%, U.S.P. | 2,500 |
| buffer | q.s. |
| water | q.s. to 5 ml |

The ingredients are mixed in conventional manner and filled into an individual container, to provide an oral solution comprising the equivalent of 50 or 100 mg free (±)-COMPOUND, suitable for use in accordance with the present invention, e.g. for administration as one of a series of individual doses administered at a rate of 1 - 3x daily.

Dosages of COMPOUND, e.g. of (±)-COMPOUND or (-)-COMPOUND, employed in practicing the method of the present invention, as well as the active ingredient content of dosage forms, in particular unit dosage forms, for use in practicing the method of the present invention, are determined by the particular and novel utility taught. Moreover specific dosage forms for use in practicing the method of the present invention, in particular dosage forms for administration by inhalation in particulate, especially liquid particuate, form to the surfaces of the lungs by application from an appropriate spray dispenser device, e.g. an aerosol, atomiser, nebulizor or the like, are both specifically required by, and unique to, utility of COMPOUND as taught by the present invention. Such forms are neither known from nor suggested by the teachings of the art in relation to COMPOUND. In so far as such compositions, in particular in dosage form for inhalation or in dosage form, in particular unit dosage form, containing COMPOUND in specified amount are novel (whether,

in the latter case, as lying outside of the teachings of the art or as selections from within the broad teachings of the art), these also form an integral part of the present invention.

Accordingly, in a further series of specific embodiments, the present invention also provides:

1. A pharmaceutical composition (e.g. for use in the treatment of obstructive or inflammatory airways disease and/or disease characterised by, or having an aetiology comprising, morbid eosinophil accumulation, for example for use in accordance with any of the specific methods hereinbefore defined, in particular for the treatment of asthma, pneumoconiosis, hypereosinophilia, eosinophil-related conditions or bronchitis), in unit dosage form and comprising COMPOUND as active ingredient in an amount of from 0.75 to 800, e.g. from 0.75 to 100 or to 300, mg/unit dosage, together with one or more pharmaceutically acceptable diluents or carriers therefor:

2. A pharmaceutical composition according to 1, for oral administration:

3. A pharmaceutical composition according to 1 or 2 comprising (±)-COMPOUND as active ingredient in an amount of from 1.5 to 800, e.g. from 1.5 to 100 or to 300, mg/unit dosage; suitably from 5 to 100 mg/unit dosage:

4. A pharmaceutical composition according to 1 or 2 comprising (-)-COMPOUND in pure or substantially pure form as active ingredient in an amount of from 0.75 to 400, e.g. from 0.75 to 50 or to 150, mg/unit dosage; suitably from 0.25 to 50 mg/unit dosage:

5. A pharmaceutical composition according to 3 or 4 for bronchodilator use or for use in initiating therapy, e.g. when used in a method of treatment as set forth under 1. above, comprising (±)-COMPOUND as active ingredient in an amount of from 5 to 50 mg/unit dosage or (-)-COMPOUND in pure or substantially pure form as active ingredient in an amount of from 2.5 to 25 mg/unit dosage:

6. A pharmaceutical composition according to 3 or 4 for prophylactic use or for use in maintainance therapy, e.g. when used in a method of treatment as defined under 1 above, comprising (±)-COMPOUND as active ingredient in an amount of from 1.5 to 30 mg/unit dosage or (-)-COMPOUND in pure or substantially pure form as active ingredient in an amount of from 0.5 to 15 mg/unit dosage:

7. A pharmaceutical composition according to any one of 1 to 6 above wherein COMPOUND is present in an amount of less than 3.0 mg/unit dosage or more than 400 mg/unit dosage:

8. A pharmaceutical composition in liquid form and suitable or adapted for administration in spray or other liquid particulate form to the internal sufaces of the lung and comprising COMPOUND as active ingredient together with one or more pharmaceutically acceptable liquid diluents or carriers therefor:

9. A process for the preparation of a pharmaceutical composition as defined under any one of 1 to 8 above which process comprises intimately admixing COMPOUND together with one or more pharmaceutically acceptable diluents or carriers therefor: as well as

10. COMPOUND for use in the preparation of a composition in accordance with any one of 1 to 8 above, e.g. for use in a method of treatment as set forth under 1 above.

Suitable unit dosage forms as defined under 1 through 7 above comprise, e.g. tablets and capsules, for example as hereinbefore described.

Compositions as defined under 8 above may be prepared in essentially conventional manner. Such compositions include e.g. solutions or fine dispersions of COMPOUND, e.g. in pharmaceutically acceptable acid addition salt form (e.g. as the bis-hydrogen maleate or bis-hydrochloride), in particular aqueous solutions or dispersions. Such solutions or dispersions will also suitably comprise one or more surface-active agents, e.g. to promote or assist nebulization or distribution upon or adsorption at the lung surface following inhalation. Such compositions will suitably be contained in an appropriate delivery device, e.g. aerosol, nebulizor or atomizer. Such device will preferably be adapted to administer a fixed spray volume on single or multiple, e.g. double or triple, activation, the total amount of COMPOUND delivered in said fixed spray volume being, e.g. for the purposes of asthma therapy, suitably of the order of from 0.15 to 30 or up to 100 mg, for example, in the case of (±)-COMPOUND in pure or substantially pure form, of the order of from 0.3 to 100 mg or less (e.g. from 0.3 up to 15, 30 or 50 mg) or, in the case of (-)-COMPOUND in pure or substantially pure form, of the order of from 0.15 to 50 mg or less (e.g. from 0.15 up to 7.5, 15 or 25 mg), or otherwise as hereinbefore indicated.

Such device will also suitably be provided with means to facilitate administration of contained composition by inhalation, e.g. as known in the art for other medication applied by inhalation to the internal surfaces of the lungs. Such means may, for example, include provision of an outlet (e.g. nozzle) to said container, shaped or adapted for application into or at the oral orifice, ensuring or easing inhalation of composition exiting through said outlet on acuation of the device.

CLAIMS

1. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the treatment of obstructive or inflammatory airways disease.

2. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use as a bronchodilator.

3. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the prophylactic treatment of inflammatory airways disease.

4. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the suppression of eosinophil accumulation and/or activation.

5. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the treatment of asthma.

6. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the treatment of pneumoconiosis.

7. The use of cis-6-(4-acetanilido)-8,9-dimethoxy-2-methyl-1,2,3,4,4a,10b-hexahydro-benzo[c][1,6]naphthyridine in the preparation of a pharmaceutical composition for use in the treatment of hypereosinophilia, eosinophil-related disorder or atopy.

8. The use in accordance with any one of claims 1 to 8 of (±)-cis-6-(4-acetanilido)-8,9-dimethoxy-2-methyl-1,2,3,4,-4a,10b-hexahydro-benzo[c][1,6]naphthyridine or of (-)-cis-6-(4-acetanilido)-8,9-dimethoxy-2-methyl-1,2,3,4,4a,10b-hexahydro-benzo[c][1,6]naphthyridine in pure or substantially pure form.

9. The use in accordance with any one of claims 1 to 9 wherein the cis-6-(4-acetanilido)-8,9-dimethoxy-2-methyl-1,2,3,4,-4a,10b-hexahydro-benzo[c][1,6]naphthyridine is in pharmaceutically acceptable acid addition salt, for example bis-hydrogen maleate or bis-hydrochloride, form.

10. A pharmaceutical composition: in unit dosage form and comprising cis-6-(4-acetanilido)-8,9-dimethoxy-2-methyl-1,2,3,4,4a,10b-hexahydro-benzo[c][1,6]naphthyridine as active ingredient in an amount of from 0.75 to 800 mg/unit dosage, together with one or more pharmaceutically acceptable diluents or carriers therefor; or in liquid form and suitable or adapted for administration in spray or other liquid particulate form and comprising cis-6-(4-acetanilido)-8,9-dimethoxy-2-methyl-1,2,3,4,4a,10b-hexahydro-benzo-[c][1,6]-naphthyridine as active ingredient, together with one or more pharmaceutically acceptable diluents or carriers therefor.